Europäisches Patentamt

European Patent Office

Office européen des brevets

(11) Publication number: **0 533 820 B1**

(12) **EUROPEAN PATENT SPECIFICATION**

(45) Date of publication of patent specification: **09.11.94**　(51) Int. Cl.⁵: **A61K 9/06**

(21) Application number: **91912105.3**

(22) Date of filing: **12.06.91**

(86) International application number:
**PCT/DK91/00156**

(87) International publication number:
**WO 91/19480 (26.12.91 91/29)**

(54) **PHARMACEUTICAL PREPARATION.**

(30) Priority: **19.06.90 DK 1487/90**

(43) Date of publication of application:
**31.03.93 Bulletin　93/13**

(45) Publication of the grant of the patent:
**09.11.94 Bulletin　94/45**

(84) Designated Contracting States:
**AT BE CH DE DK ES FR GB GR IT LI LU NL SE**

(56) References cited:
**EP-A- 0 261 599**
**EP-A- 0 308 238**
**EP-A- 0 330 905**

(73) Proprietor: **NOVO NORDISK A/S**
**Novo Allé**
**DK-2880 Bagsvaerd (DK)**

(72) Inventor: **Hoelgaard, Annie**
**Kastanievej 35**
**DK-2840 Holte (DK)**

(74) Representative: **Nilausen, Kim**
**c/o Novo Nordisk A/S**
**Novo Allé**
**DK-2880 Bagsvaerd (DK)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

**Description**

DETAILED DESCRIPTION OF THIS INVENTION

This invention relates to water soluble compressed freeze-dried preparations containing a wound-healing medicament and a base material. Thus, this invention is directed to a pharmaceutical formulation designed for topical administration.

BACKGROUND OF THIS INVENTION

There are a variety of formulation types available for medicated topical therapy in wound management. There are solutions, powders, fluid suspensions or emulsions and semisolids. Semisolids such as creams, ointments, pasts and gels fulfil a special topical need in that they are able to cling to the application surface for an extended period, generally until they are washed out by the wound exudate or washed off by cleaning the wound area. Likewise, solutions have poor staying properties. Drugs or medicaments can be applied onto the wound by impregnation of or incorporation into dressings, swabs, films or gauze which are in contact with the wound tissue.

Freeze drying (also designated lyophilisation) is a well known process of drying. Lyophilisation of mixtures containing a medicament is known. The reasons for lyophilizing such mixtures are to obtain a stable preparation and to provide a long shelf life. The resulting product can be designated a lyophilisate.

The normal way of using such lyophilisates is to dissolve it in a solvent, normally water, prior to use. This procedure is called a reconstitution. The solution obtained constitutes the medicament which the patient is using like any other similar medicament.

An example of such a lyophilized formulation is described in European patent application having the publication No. 308,238. Example 7 therein describes a lyophilisate containing hydroxypropylmethyl cellulose and epidermal growth factor.

It has for some time been recognized that a number of tropic factors, due to their stimulation of cell growth and differentiation, may have an enhancing effect on wound healing.

Several medicaments are known as having a wound healing effect. For example, human growth hormone (herein-after designated hGH) being a superior pituitary gland hormone is known to have a wound healing effect. hGH can be administered topically. The effect of hGH on wound healing is thought to be mediated through growth factors. Specific growth hormone receptors on monocytes have been found. hGH may affect the monocytes to paracrine delivery of the well-known growth factors, i.e., PDGF, EGF, TGF-$\alpha$ and bFGF. Another explanation could be that hGH affects the fibroblasts to autocrine delivery of IGF-I. IGF-I has been shown to exhibit mitogen effect on fibroblasts.

The object of this invention is to make available a medical formulation which conveniently can be used as a wound healing preparation.

DETAILED EXPLANATION OF THIS INVENTION

It has, surprisingly, been found that if a lyophilisate containing a wound healing medicament is compressed to a certain degree, a preparation which conveniently can be placed directly on or into the wound is obtained.

The lyophilisate is obtained by freeze drying a solution or suspension of a base material and a medicament having a wound healing effect. The content of medicament in the lyophilisate is conveniently in the range from about 0.1 - 10 percentage (weight/weight). Thereafter, the lyophilisate is compressed so as to obtain a soft and flexible product which, when placed on the wound, does not or only to a minor degree annoy the patient. Conveniently, the lyophilisate is compressed to from about 1/2 to about 1/10 of its original volume, preferably, to from about 1/3 to about 1/6. The density of the compressed lyophilisates according to this invention is, preferably, in the range from 50 to 200 mg/cm$^3$, more preferred in the range from 60 to 120 mg/cm$^3$. The thickness of the compressed lyophilisates according to the invention is preferably in the range from 0.2 to 1.5 mm.

The base material present in the compressed lyophilisates according to this invention is conveniently a gelling agent.

An important aspect of the present invention is the use of an appropriate gelling agent in the dermatologically acceptable vehicle. The gelling agent used must be chemically inert in compositions with the medicament. Therefore, it must be non-ionic. Additionally, since the gel is to be prepared prior to lyophilization, the gelling agent must dissolve completely to produce a transparent gel. Additionally, the gelling agent used must allow for the hydrogel to release a sufficient amount of medicament within a few hours. Additionally, the gelling agent must form a hydrogel which allows for the generation of a coherent porous freeze-dried matrix by lyophilization.

Gelling agents possessing these qualities are specific cellulose ether compounds, preferably hydroxyethylcellulose, hydroxypropylcellulose hydroxypropylmethylcellulose, hydroxyethylmethylcellulose and methylcellulose, most preferred hydroxyethylcellulose (hereinafter designated HEC).

The material of this invention may be prepared by:

a) forming a liquid hydrogel containing the medicament dissolved therein, the viscosity of the hydrogel preferably being in the range from 500 to 10,000 mPas (cps), most preferred in the range from 1000 to 4000 mPas (cps),

b) freeze drying the solution in a layer to form a freeze-dried matrix containing the medicament homogenously dispersed therein, and

c) applying pressure to the matrix to form a flexible sheet, where the medicament is in an effective dosage concentration per $cm^2$.

The wound healing materials of this invention are soft flexible sheets having a porous structure.

It is very convenient for the patient that the compressed lyophilisates can be placed directly on the wound. Hence, the patient does not have to make a reconstitution of the lyophilisate. In addition, it is possible to prepare compressed lyophilisates from which pieces of a desired size can be cut or punched out to be placed on the wound.

It has been found that in the presence of moisture, some medicaments, for example hGH, loose biological activity during storage. Such loss of activity makes it impractical to store aqueous dermatological preparations containing such medicaments, for example hGH. This invention provides means for preventing loss of activity by providing stable lyophilized formulations containing the medicament means. This invention also provides means for preparing a composition comprising a gelling agent which is biologically, chemically and physically compatible with the medicament.

Heretofore, hGH has not been lyophilized with a gelling agent and an extender to prepare a product suitable for use in wound healing application.

Another important aspect of the present invention is the use of polyethylene glycols (hereinafter designated PEG) in the vehicle. PEG acts as a bulk agent which imposes softness and smoothness to the freeze-dried sheet. PEG is dermatologically acceptable and chemically inert in compositions comprising various medicaments, for example human growth hormone. Additionally, PEG has only a minor effect on the viscosity of the hydrogel. PEG imposes softness to the freeze- dried matrix and prevents its surface from becoming icy and hard.

In order to stabilize some medicaments, for example hGH, buffer agents, cryoprotectants and a non-ionic surface active component are included in the hydrogel. Preferably, phosphate buffer (pH 7.0 - 7.5) Tween 80, mannitol and glycin are used.

According to a preferred aspect of the invention, the concentration of human growth hormone is in the range from 0.1 to 2.0 $IU/cm^2$.

The present invention describes a method to prepare soft and flexible sheets where both surfaces are smooth.

Another advantage of sheets prepared from the described method is their fast release of some medicaments, for example hGH. The sheet forms a hydrogel initially after contact with the aqueous medium and releases up to 100% of some medicaments, for example hGH, into the surrounding aqueous medium.

The flexible sheet of the invention can be combined with a backing material being a wound dressing or bandage which also forms an aspect of the invention. Most preferred, the medicated sheet sticks to an adhesive dressing producing an integrated medicated wound cover. The sheet may be combined with a hydrocolloid dressing comprising the same hydrocolloid as the material in the sheet. The hydrocolloid in the dressing as well as in the sheet swells locally in a linear fashion and expands into the wound cavity where it applies both osmotic and physical pressure to the wound bed.

The freeze-dried sheet offers a potential for combined products with hydrocolloid dressings or films where the sheet sticks to the dressing or film. Wound dressing is here used to embrace a wide spectrum of materials and includes absorbents, surgical adhesive tapes and bandages.

The invention is explained with reference to the drawings on which

Fig. 1    shows a Scanning Electron Microscopy of a lyophilized product according to the invention, and

Fig. 2    shows a Scanning Electron Microscopy as in Fig. 1 but in a greater scale.

The invention is further explained in the below examples which are presented to illustrate this invention.

EXAMPLE 1

A preferred method to prepare a smooth, soft and flexible dry sheet is as follows: A solution containing 1.0% PEG 6000 and 1.5% hydroxyethylcellulose (Natrosol Hx Pharm 250 TM) was prepared in water. B-hGH (biosynthetic hGH) was added resulting in a final concentration of 200 $\mu$g of per gram gel. The gel was placed in 5 cm diameter petri-dishes in a layer of 3 mm.

The gel was lyophilized. The dry gel was soft and homogenous. It was pressed into thin (1 mm) soft sheets and cut into pieces of appropriate sizes. In an in vitro kinetic study in tris-buffer (pH = 7.4), the sheet was found to dissolve and release 100% of its hGH during 0.5 hours (37°C).

The hydrogel prepared above was placed in 7.5 ml vials in a layer of 3 mm. The vials were placed in a lyophilization chamber and the following freeze-drying cyclus was used:

Step 1) -40°C for 4 hours at 1 atm.

Step 2) -40°C for 1 hour at full vacuum.

Step 3) -30°C for 3/4 hour at full vacuum.

Step 4) -20°C for 4 hours at full vacuum.

Step 5) 0°C for 15 hours at full vacuum.

Step 6) 30°C for 11 hours at full vacuum.

The vials were closed in the freeze-dryer at the end of the cyclus. Samples were analyzed for hGH and its polymer and dimer forms and for content of moisture.

| Batch | hGH, mg/g | polymer, % | dimer, % | $H_2O$, % |
|---|---|---|---|---|
| 91120 t = 1 month (4°C) | 18 | 0.7 | 4.2 | - |
| 909-18 t = 0 | 18 | 0.7 | 6.0 | 2.0 |

EXAMPLE 2

In order to study the stability of dry gels containing hGH, gels were freeze-dried in 7.5 ml glass vials in a layer of 3 mm as described in Example 1.

The following compositions were prepared for accelerated stability study:

| Composition | a | b | c | Control |
|---|---|---|---|---|
| hGH (IU) | 5 | 5 | 5 | 5 IU |
| Glycin | 1.3 | 1.3 | 1.3 | 1.3 mg |
| Mannitol | | | | 36 mg |
| $NaH_2PO_4 \cdot H_2O$ | .7 | .7 | .7 | .7 mg |
| $Na_2HPO_4 \cdot 12H_2O$ | 5.3 | 5.3 | 5.3 | 5.3 mg |
| PEG 6000 | | | 8.0 | mg |
| Tween 80 | | 2.0 | 2.0 | mg |
| Natrosol Hx250 | 24 | 24 | 24 | mg |

The vials were stored at 25°C and 37°C for 2, 4 and 8 weeks.

The results are shown in the below Table.

| TIME | TEMP. | COMPOSITION | hGH IU | DESAMIDO % | POLYMER % | DIMER % | H₂O % |
|---|---|---|---|---|---|---|---|
| 0 | - | a | 4.6 | 4.5 | <0.5 | 1.4 | 3.5 |
| | | b | 4.8 | 4.4 | <0.5 | 2.0 | 3.1 |
| | | c | 5.2 | 4.5 | <0.5 | - | 2.8 |
| | | control | 5.6 | 4.2 | 0.7 | 2.3 | 1.3 |
| 2 weeks | 25 C | a | 5.0 | 3.9 | 0.5 | 2.3 | |
| | | b | 4.2 | 4.0 | <0.5 | 2.2 | |
| | | c | 4.2 | 3.6 | <0.5 | 2.9 | |
| | | control | 5.8 | 3.9 | 0.6 | 1.5 | |
| | 37 C | a | 4.4 | 4.8 | <0.5 | 3.5 | |
| | | b | 4.8 | 4.7 | <0.5 | 3.1 | |
| | | c | 4.6 | 4.2 | <0.5 | 4.1 | |
| | | control | 5.4 | 3.5 | 0.8 | 5.7 | |
| 4 weeks | 25 C | a | - | 5.0 | 0.7 | 2.3 | |
| | | b | - | 3.4 | <0.5 | 2.2 | |
| | | c | - | 3.6 | <0.5 | 2.9 | |
| | | control | - | 1.7 | 0.5 | 1.5 | |
| | 37 C | a | 4.6 | 6.4 | <0.5 | 3.9 | |
| | | b | 4.2 | 4.1 | <0.5 | 4.1 | |
| | | c | 4.6 | 5.3 | <0.5 | 6.1 | |
| | | control | 4.2 | 2.9 | 0.8 | 8.0 | |
| 8 weeks | 25 C | a | - | 3.4 | 1.0 | 2.2 | |
| | | b | - | 3.8 | <0.5 | 2.6 | |
| | | c | - | 3.6 | <0.5 | 2.8 | |
| | | control | - | 3.4 | 1.5 | 2.7 | |
| | 37 C | a | 4.8 | 7.9 | <0.5 | 3.6 | |
| | | b | 4.8 | 6.1 | <0.5 | 4.7 | |
| | | c | 4.6 | 6.5 | <0.5 | 6.3 | |
| | | control | 4.6 | 6.6 | 0.8 | 8.8 | |

The data show that the dry gels containing hGH are stable and that hGH in the freeze-dried gels has a stability similar to that of the control which is a conventional composition for parenteral use.

5

EXAMPLE 3

This example illustrates that hydroxyethyl cellulose is compatible with hGH for the purpose of the present invention.

The below preparation containing hGH and 0.6% hydroxyethylcellulose in water was tested for biological activity by the Tibia test in rats.

A hydrogel consisting of the following components was prepared:

| | |
|---|---|
| hGH (Norditropin™), 4 IU/ml: | 1000 $\mu$l and 500 $\mu$l. |
| sodium chloride solution (0.9%): hydroxyethylcellulose | 9.00 ml. |
| (Natrosol Hx Pharm 250), 1.2%: | 10.00 ml. |

According to the result from the tibia test, the biological activity of hGH in this hydrogel was maintained.

An in vitro hGH-receptor assay was performed by comparing an aqueous composition containing 1.2% hydroxyethylcellulose with a control composition without the gelling agent. The cell used was IM-9 human cell line. The assay is based on competition between a known labelled hGH ($^{125}$I-hGH) and the unknown hGH sample. The measured radioactivity is then related to a constructed standard curve.

1. hGH: 12 IU/ml in water (control).

2. hGH: 12 IU/ml in 1.2% Natrosol Hx Pharm in water.

There was no significant difference in activity between the two compositions.

EXAMPLE 4

In this example physical properties of two compressed dry gel products are characterized.

Dry gels were prepared by freeze drying hydrogel solutions of the following compositions:

| | A | B |
|---|---|---|
| hGH | .05 | .05 % |
| Natrosol HX250 Pharm. | 1.5 | 1.5 % |
| PEG 6000 | 1.0 | 1.0 % |
| Glycin | | 0.5 % |
| Tween 80 | | .5 % |
| Mannitol | | .5 % |
| $Na_2HPO_4.2H_2O$ | | .19 % |
| $NaH_2PO_4.H_2O$ | | .037 % |
| Sterile water ad | 100 | 100 % |

27 g hydrogel solution were filled into 9 cm petri dishes and lyophilized.

The derived dry gels had a hight of 4 mm. They were punsched into dics of 10 mm diameter.

Test of tensile strength was performed according to DIN 53455:

Gel type A: 0.093 N/mm$^2$      B: 0.095 N/mm$^2$

Test of pressure resistance was performed according to DIN 534553:

| Gel type A reduction of hight (%) | Gel type B reduction of hight (%) | force applied (N/mm$^2$) |
|---|---|---|
| 5 | 7.5 | .005 |
| 8.8 | 18 | .01 |
| 20 | 49 | .02 |
| 43 | 65 | .03 |
| 58 | 71 | .04 |

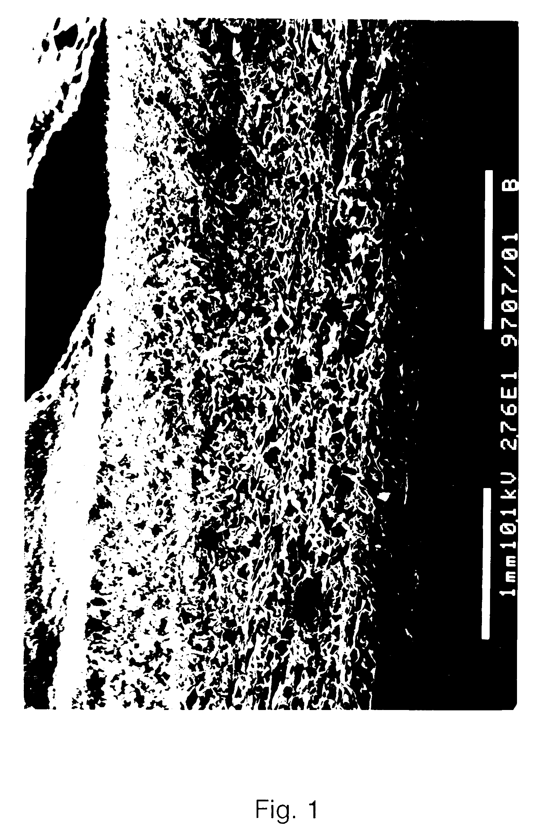

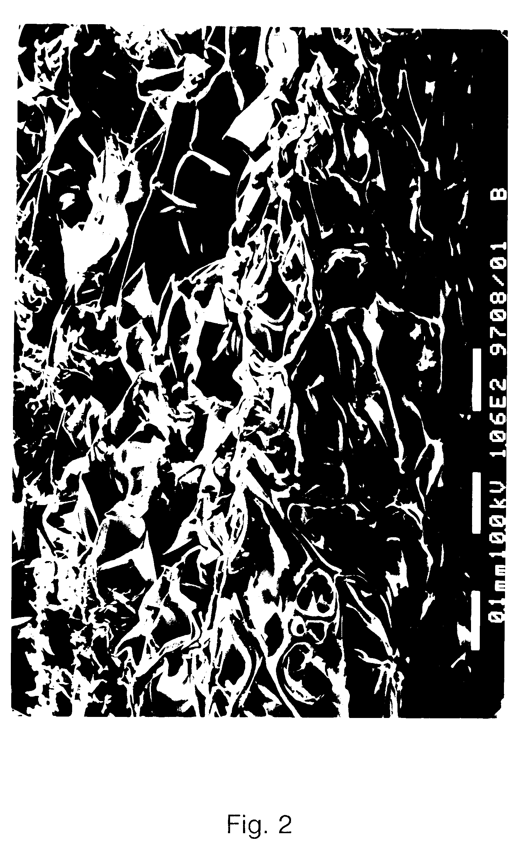

Scanning Electron Microscopy of the compressed freeze-dried product prepared from gel type A was performed. A cross-section of a disc was examined and the porous structure of the sheet is clearly shown by ESM-pictures in Figures 1 and 2.

**Claims**
**Claims for the following Contracting States : AT, BE, CH, LI, DE DK, FR, GB, IT, LU, NL, SE**

1. Water soluble wound healing material, characterized in that it is in the form of a compressed freeze-dried product containing a wound healing medicament, a base material in the form of a gelling agent and a polyethylene glycol, wherein the freeze-dried product is compressed to from about 1/2 to about 1/10 of its original volume.

2. Material according to claim 1 characterized in that the medicament is human growth hormone.

3. Material according to claim 1 or 2 characterized in that the base material is a hydroxycellulose ether, preferably hydroxyethylcellulose, hydroxymethylpropylcellulose hydroxypropylcellulose, methylcellulose or hydroxyethylmethylcellulose, preferably hydroxyethylcellulose.

4. Material according to any one of the preceding claims characterized in that it contains PEG having a molecular weight in the range from 3000 to 20,000, preferably from 6000 to 8000.

5. Material according to any one of the preceding claims characterized in that it contains a buffer agent.

6. Material according to any one of the preceding claims characterized in that it contains a non-ionic surface active component and optionally a water soluble extender for lyophilization.

7. Material according to any one of the preceding claims, characterized in that the concentration of human growth hormone is in the range from 0.1 to 2.0 IU/cm$^2$.

8. Material according to any one of the preceding claims, characterized in that the concentration of hydroxycellulose ether is in the range from 20 to 90%, preferably from 30 to 75% (weight/weight).

9. Material according to any one of the preceding claims, characterized in that the concentration of PEG is in the range from 0 to 70%, preferably from 15 to 50% (weight/weight).

10. Material according to any one of the preceding claims, characterized in that the flexible sheet is porous and has a density larger than about 0.05 g/cm$^3$.

**Claims for the following Contracting States : ES, GR**

1. A method for producing a water soluble wound healing material in the form of a compressed freeze-dried product containing a wound healing medicament, a base material in the form of a gelling agent and a polyethylene glycol, characterized in that a lyophilisate is obtained by freeze drying a solution or suspension of the base material and the medicament having a wound healing effect whereafter the freeze-dried product is compressed to from about 1/2 to about 1/10 of its original volume.

2. A method according to claim 1 characterized in that the medicament is human growth hormone.

3. A method according to claim 1 or 2 characterized in that the base material is a hydroxycellulose ether, preferably hydroxyethylcellulose, hydroxymethylpropylcellulose hydroxypropylcellulose, methylcellulose or hydroxyethylmethylcellulose, preferably hydroxyethylcellulose.

4. A method according to any one of the preceding claims characterized in that PEG having a molecular weight in the range from 3000 to 20,000, preferably from 6000 to 8000 is used.

5. A method according to any one of the preceding claims characterized in that a buffer agent is added.

6. A method according to any one of the preceding claims characterized in that a non-ionic surface active component and optionally a water soluble extender for lyophilization is added.

7. A method according to any one of the preceding claims, characterized in that the concentration of human growth hormone is in the range from 0.1 to 2.0 IU/cm$^2$.

7

8. A method according to any one of the preceding claims, characterized in that the concentration of hydroxycellulose ether is in the range from 20 to 90%, preferably from 30 to 75% (weight/weight).

9. A method according to any one of the preceding claims, characterized in that the concentration of PEG is in the range from 0 to 70%, preferably from 15 to 50% (weight/weight).

10. A method according to any one of the preceding claims, characterized in that the flexible sheet produced is porous and has a density larger than about 0.05 g/cm$^3$.

**Patentansprüche**
**Patentansprüche für folgende Vertragsstaaten : AT, BE, CH, LI, DE, DK, FR, GB, IT, LU, NL, SE**

1. Wasserlösliches Wundheilungsmaterial, dadurch gekennzeichnet, daß es in Form eines verpreßten gefriergetrockneten Produkts vorliegt, das ein wundheilendes Arzneimittel, ein Grundmaterial in Form eines Gelbildungsmittels sowie ein Polyethylenglycol enthält, wobei das gefriergetrocknete Produkt auf etwa 1/2 bis 1/10 seines ursprünglichen Volumens verpreßt ist.

2. Material nach Anspruch 1, dadurch gekennzeichnet, daß das Arzneimittel menschliches Wachstumshormon ist.

3. Material nach Anspruch 1 oder 2, dadurch gekennzeichnet, daß das Grundmaterial ein Hydroxycelluloseether, vorzugsweise Hydroxyethylcellulose, Hydroxymethylpropylcellulose, Hydroxypropylcellulose, Methylcellulose oder Hydroxyethylmethylcellulose und vorzugsweise Hydroxyethylcellulose, ist.

4. Material nach einem der vorhergehenden Ansprüche, dadurch gekennzeichnet, daß es PEG mit einem Molekulargewicht im Bereich von 3000 bis 20000 und vorzugsweise von 6000 bis 8000 enthält.

5. Material nach einem der vorhergehenden Ansprüche, dadurch gekennzeichnet, daß es ein Puffermittel enthält.

6. Material nach einem der vorhergehenden Ansprüche, dadurch gekennzeichnet, daß es ein nichtionisches grenzflächenaktives Mittel als Komponente sowie wahlweise ein wasserlösliches Streckmittel zur Lyophilisierung enthält.

7. Material nach einem der vorhergehenden Ansprüche, dadurch gekennzeichnet, daß die Konzentration des menschlichen Wachstumshormons im Bereich von 0,1 bis 2,0 IU/cm$^2$ liegt.

8. Material nach einem der vorhergehenden Ansprüche, dadurch gekennzeichnet, daß die Konzentration des Hydroxycelluloseethers im Bereich von 20 bis 90 % und vorzugsweise im Bereich von 30 bis 75 % (Gewicht/Gewicht) liegt.

9. Material nach einem der vorhergehenden Ansprüche, dadurch gekennzeichnet, daß die Konzentration an PEG im Bereich von 0 bis 70 % und vorzugsweise im Bereich von 15 bis 50 % (Gewicht/Gewicht) liegt.

10. Material nach einem der vorhergehenden Ansprüche, dadurch gekennzeichnet, daß das flexible Flachmaterial porös ist und eine Dichte von mehr als etwa 0,05 g/cm$^3$ aufweist.

**Patentansprüche für folgende Vertragsstaaten : ES, GR**

1. Verfahren zur Herstellung eines wasserlöslichen Wundheilungsmaterials in Form eines verpreßten gefriergetrockneten Produkts, das ein wundheilendes Arzneimittel, ein Grundmaterial in Form eines Gelbildungsmittels sowie ein Polyethylenglycol enthält, dadurch gekennzeichnet, daß durch Gefriertrocknen einer Lösung oder Suspension des Grundmaterials und des wundheilenden Arzneimittels ein Lyophilisat hergestellt wird, worauf das gefriergetrocknete Produkt auf etwa etwa 1/2 bis etwa 1/10 seines ursprünglichen Volumens verpreßt wird.

**2.** Verfahren nach Anspruch 1, dadurch gekennzeichnet, daß das Arzneimittel menschliches Wachstumshormon ist.

**3.** Verfahren nach Anspruch 1 oder 2, dadurch gekennzeichnet, daß das Grundmaterial ein Hydroxycelluloseether, vorzugsweise Hydroxyethylcellulose, Hydroxymethylpropylcellulose, Hydroxypropylcellulose, Methylcellulose oder Hydroxyethylmethylcellulose, und vorzugsweise Hydroxyethylcellulose ist.

**4.** Verfahren nach einem der vorhergehenden Ansprüche, dadurch gekennzeichnet, daß PEG mit einem Molekulargewicht im Bereich von 3000 bis 20000 und vorzugsweise im Bereich von 6000 bis 8000 verwendet wird.

**5.** Verfahren nach einem der vorhergehenden Ansprüche, dadurch gekennzeichnet, daß ein Puffermittel zugesetzt wird.

**6.** Verfahren nach einem der vorhergehenden Ansprüche, dadurch gekennzeichnet, daß ein nichtionisches grenzflächenaktives Mittel als Komponente sowie wahlweise ein wasserlösliches Streckmittel zur Lyophilisierung zugesetzt werden.

**7.** Verfahren nach einem der vorhergehenden Ansprüche, dadurch gekennzeichnet, daß die Konzentration des menschlichen Wachstumshormons im Bereich von 0,1 bis 2,0 IU/cm$^2$ liegt.

**8.** Verfahren nach einem der vorhergehenden Ansprüche, dadurch gekennzeichnet, daß die Konzentration des Hydroxycelluloseethers im Bereich von 20 bis 90 % und vorzugsweise im Bereich von 30 bis 75 % (Gewicht/Gewicht) liegt.

**9.** Verfahren nach einem der vorhergehenden Ansprüche, dadurch gekennzeichnet, daß die Konzentration an PEG im Bereich von 0 bis 70 % und vorzugsweise im Bereich von 15 bis 50 % (Gewicht/Gewicht) liegt.

**10.** Verfahren nach einem der vorhergehenden Ansprüche, dadurch gekennzeichnet, daß das hergestellte flexible Flachmaterial porös ist und eine Dichte von mehr als etwa 0,05 g/cm$^3$ aufweist.

**Revendications**
**Revendications pour les Etats contractants suivants : AT, BE, CH, LI, DE, DK, FR, GB, IT, LU**

**1.** Matière hydrosoluble pour la guérison de plaies, caractérisée en ce qu'elle se présente sous la forme d'un produit lyophilisé comprimé contenant un principe actif pour la guérison de plaies, une matière de base sous forme d'un agent de gélification et un polyéthylène-glycol, le produit lyophilisé étant comprimé à environ 1/2 jusqu'à environ 1/10 de son volume d'origine.

**2.** Matière selon la revendication 1, caractérisée en ce que le médicament est une hormone de croissance humaine.

**3.** Matière selon la revendication 1 ou 2, caractérisée en ce que la matière de base est un éther d'hydroxycellulose, de préférence une hydroxyéthylcellulose, une hydroxyméthylpropylcellulose, une hydroxypropylcellulose, une méthylcellulose ou une hydroxyéthylméthylcellulose, de préférence une hydroxyéthylcellulose.

**4.** Matière selon l'une quelconque des revendications précédentes, caractérisée en ce qu'elle contient du PEG ayant une masse moléculaire dans la plage de 3000 à 20000 de préférence de 6000 à 8000.

**5.** Matière selon l'une quelconque des revendications précédentes, caractérisée en ce qu'elle contient un agent tampon.

**6.** Matière selon l'une quelconque des revendications précédentes, caractérisée en ce qu'elle contient un composant tensioactif nonionique et facultativement un diluant hydrosoluble pour lyophilisation.

**7.** Matière selon l'une quelconque des revendications précédentes, caractérisée en ce que la concentration de l'hormone de croissance humaine se situe dans la plage de 0,1 à 2,0 UI/cm$^2$.

**8.** Matière selon l'une quelconque des revendications précédentes, caractérisée en ce que la concentration d'hydroxycellulose éther se situe dans la plage de 20 à 90%, de préférence de 30 à 75% poids sur poids.

**9.** Matière selon l'une quelconque des revendications précédentes, caractérisée en ce que la concentration de PEG se situe dans la plage de 0 à 70%, de préférence de 15 à 50% (poids sur poids).

**10.** Matière selon l'une quelconque des revendications précédentes, caractérisée en ce que la feuille souple et poreuse présente une densité supérieure à environ 0,05g/cm$^3$.

**Revendications pour les Etats contractants suivants : ES, GR**

**1.** Procédé hydrosoluble pour la guérison de plaies, caractérisé en ce qu'il se présente sous la forme d'un produit lyophilisé comprimé contenant un principe actif pour la guérison de plaies, une matière de base sous forme d'un agent de gélification et un polyéthylène-glycol, le produit lyophilisé étant comprimé à environ 1/2 jusqu'à environ 1/10 de son volume d'origine.

**2.** Procédé selon la revendication 1, caractérisé en ce que le médicament est une hormone de croissance humaine.

**3.** Procédé selon la revendication 1 ou 2, caractérisé en ce que la procédé de base est un éther d'hydroxycellulose, de préférence une hydroxyéthylcellulose, une hydroxyméthylpropylcellulose, une hydroxypropylcellulose, une méthylcellulose ou une hydroxyéthylméthylcellulose, de préférence une hydroxyéthylcellulose.

**4.** Procédé selon l'une quelconque des revendications précédentes, caractérisé en ce qu'il contient du PEG ayant une masse moléculaire dans la plage de 3000 à 20000 de préférence de 6000 à 8000.

**5.** Procédé selon l'une quelconque des revendications précédentes, caractérisé en ce qu'il contient un agent tampon.

**6.** Procédé selon l'une quelconque des revendications précédentes, caractérisé en ce qu'il contient un composant tensioactif nonionique et facultativement un diluant hydrosoluble pour lyophilisation.

**7.** Procédé selon l'une quelconque des revendications précédentes, caractérisé en ce que la concentration de l'hormone de croissance humaine se situe dans la plage de 0,1 à 2,0 UI/cm$^2$.

**8.** Procédé selon l'une quelconque des revendications précédentes, caractérisé en ce que la concentration d'hydroxycellulose éther se situe dans la plage de 20 à 90%, de préférence de 30 à 75% poids sur poids.

**9.** Procédé selon l'une quelconque des revendications précédentes, caractérisé en ce que la concentration de PEG se situe dans la plage de 0 à 70%, de préférence de 15 à 50% (poids sur poids).

**10.** Procédé selon l'une quelconque des revendications précédentes, caractérisé en ce que la feuille souple et poreuse présente une densité supérieure à environ 0,05g/cm$^3$.

Fig. 1

Fig. 2